# EUROPEAN PATENT APPLICATION

(11) **EP 4 400 045 A1**
(43) Date of publication of application: **17.07.2024**
(21) Application number: 23305035.0
(22) Date of filing: 11.01.2023
(51) Int. Cl.: A61B 5/113, A61B 5/00, A61B 5/01

(54) **ASSEMBLY OF A SENSOR AND A CONDUCTIVE YARN AND WEARABLE ARTICLE INCLUDING THE ASSEMBLY**

(71) Applicant: CHRONOLIFE, 75012 Paris (FR)
(72) Inventor: Roger, Harmony, 77173 CHEVRY COSSIGNY (FR); ROMAO, Fernando, 75016 PARIS (FR)
(74) Representative: Plasseraud IP

(57) **Abstract**

The disclosure refers to an assembly (101) comprising at least:
- a conductive yarn (110); and
- a physiological sensor having electrically conductive properties,
wherein the conductive yarn (110) is knotted to the elongation sensor (102) in a knotted portion (112) to provide electrical contact between the conductive yarn (110) and the elongation sensor (102).

## Description

The present disclosure generally relates to the field of assemblies of physiological sensors and conductive yarns, and, in particular, relates to an assembly of an elongation sensor, and a conductive yarn, and to a wearable article comprising such an assembly. It further relates to an assembly of a temperature sensor, and a conductive yarn, and to a wearable article comprising such an assembly.

### BACKGROUND OF THE INVENTION

Electronic devices for detecting and processing biometric signals of a user have been developed that allow medical supervision of the user during his or her usual activities or situations. Sensors, called physiological sensors, may be applied to the skin or a specific body area, for example the chest, of the user so as to perform temperature measurements, to determine physiological parameters or to detect electrical signals related for example to a cardiac function. Monitoring the various signals delivered by these sensors allows determining a specific physiological condition of a user that might be impaired. For example, when an individual is having a seizure, specific signal features appear on the signals corresponding to the electrocardiogram (ECG) or to respiration.

For example, it is possible to permanently monitor respiratory activity by integrating an elongation sensor into a garment or some other wearable article. Such an elongation sensor may acquire information about parameters such as frequency and amplitude of breathing of a user and transmit it to external medical equipment, such as monitoring, diagnostic, or stimulating devices.

It is also possible to monitor the temperature of a part of the body of a user by integrating temperature sensors into a wearable article such as for example a garment. Such temperature sensors may acquire information about temperature and temperature changes of a user's body.

The information acquired by such physiological sensors may be transmitted via conductive yarn to an electronic board, and then further to external medical equipment, such as monitoring, diagnostic, or stimulating devices.

Assembling the physiological sensor and the conductive yarn can be complex. In particular, the connection region of the physiological sensor and the conductive yarn can be fragile. Furthermore, physiological sensors are often fabricated from materials that do not adhere to other materials, which complicates any connection of such physiological sensors to conductive yarns.

Moreover, the connection region of the physiological sensor to the conductive yarn in a wearable article is preferably waterproof, in order to be fully operative even when a wearer is sweating and after washing of the wearable article.

WO 2021/048149 A1 discloses an elongation sensor for detecting breathing of a user, and a garment comprising such an elongation sensor. According to the disclosure, the conductive yarns may be stitched to the ends of the elongation sensor, in order to electrically connect the elongation sensor to an electronic board in the garment.

### SUMMARY OF THE INVENTION

An assembly is disclosed, comprising at least:
- a conductive yarn; and
- a physiological sensor having electrically conductive properties.

According to preferred embodiment, the physiological sensor is selected in the group consisting of elongation sensor and temperature sensor.

According to one embodiment, the physiological sensor is an elongation sensor and the conductive yarn is knotted to the elongation sensor in a knotted portion to provide electrical contact between the conductive yarn and the elongation sensor.

The knotted portion formed by the conductive yarn and the elongation sensor may establish a stable, robust, compact and electrically conductive connection between the conductive yarn and the elongation sensor.

The knotted portion may comprise at least a double knot that comprises at least two superposed knots. Preferably, the knotted portion comprises a triple knot that comprises three superposed knots. The knotted portion may also comprise a quadruple knot that comprises four superposed knots, or even more.

In the knotted portion, the elongation sensor and the conductive yarn may be tied to each other and be held tight to each other.

For the sake of completeness, it is pointed out that a conductive yarn sewn or stitched to an elongation sensor does not define a knotted portion.

The elongation sensor may be a stretchable yarn whose electrically conductive properties vary when being stretched or relaxed.

The elongation sensor may be configured to be pressed against a body of a user wearing the elongation sensor, in such a way that a length of the elongation sensor varies when the user breathes or when muscles of the user are contracted or relaxed.

The conductive yarn may be a yarn having electrically conductive properties. The conductive yarn may be configured to transmit changes in the electrically conductive properties of the elongation sensor (for example to an electronic board for further analysis).

For a given force applied to the conductive yarn and to the elongation sensor, relative elongations of the conductive yarn may be small with respect to relative elongations of the elongation sensor. In addition, for a given force applied to the conductive yarn and to the elongation sensor, relative changes in the electrically conductive properties of the conductive yarn may be small when compared to relative changes of the electrically conductive properties of the elongation sensor.

In an embodiment, the assembly may further comprise an isolating wrapping layer. The isolating wrapping layer may cover the knotted portion.

The isolating wrapping layer may be an insulating material disposed on the knotted portion and adhering to the knotted portion. The isolating wrapping layer may stabilize the knotted portion, in particular when forces act on the elongation sensor. In addition, the isolating wrapping layer may protect the knotted portion against wear and humidity.

In an embodiment, the isolating wrapping layer may be attached to the knotted portion by heat-bonding.

Heat-bonding allows assembling different materials by applying heat and pressure to them. Heat-bonding allows forming a tight and stable connection between the knotted portion and the isolating wrapping layer.

In an embodiment, the knotted portion may comprise a triple knot.

A triple knot may comprise three individual knots formed between the elongation sensor and the conductive yarn. The three individual knots may be formed one above the other.

For example, one individual knot may be relatively unstable and become loose over time, in particular when the elongation sensor is made from materials that do not adhere to materials from which the conductive yarn is made.

On the contrary, the triple knot may provide a stable connection between the elongation sensor and the conductive yarn, even when the elongation sensor and the conductive yarn are made from materials that do not adhere to each other, and even when forces are applied to the elongation sensor.

In an embodiment, the elongation sensor may comprise a body of flexible material. The elongation sensor may further comprise conductive particles embedded in the body of flexible material.

The flexible material may be a material that can be bent or stretched without breaking, such as silicone. The conductive particles may be particles, for example carbon particles, that confer conductive properties to a non-conductive material when embedded in such a material.

The flexible material may confer flexible properties to the elongation sensor and allow reversible stretching and relaxing of the elongation sensor. The conductive particles may confer electrically conductive properties to the body of flexible material, that vary as a function of the elongation of the body.

In an embodiment, the conductive yarn may comprise an arrangement of chain stitches.

Compared to a conductive yarn in a linear arrangement, the arrangement of a conductive yarn in chain stitches may be more resilient, may be more easily manipulated, and may adhere better to the elongation sensor in the knotted portion.

The knotted portion may be more stable and resilient when a conductive yarn arranged in chain stitches is used.

In an embodiment, the assembly may comprise a flexible substrate. The conductive yarn may be sewn to the flexible substrate.

The flexible substrate may be made from a flexible material and provide a support for the conductive yarn. A conductive yarn sewn to a flexible substrate may be more stable and resilient when forces are applied to the conductive yarn.

In an embodiment, the conductive yarn may comprise a connection portion. The connection portion may extend beyond the flexible substrate. The connection portion of the conductive yarn may be knotted to the elongation sensor in the knotted portion.

The connection portion may be a portion of the conductive yarn intended to form the knotted portion with the elongation sensor. The connection portion may be an end of the conductive yarn or be located close to an end of the conductive yarn.

The electrical contact between the elongation sensor and the conductive yarn in the knotted portion may be optimized when using the connection portion for forming the knotted portion rather than another portion of the conductive yarn, in particular if the flexible substrate is bulky or has non-conductive properties.

According to another embodiment, the physiological sensor is a temperature sensor comprising a printed circuit board supporting a temperature-sensitive component, the conductive yarn has an end folded to form a first loop in a region providing electrical contact with the printed circuit board. In preferred embodiment, an insulating member is further placed around the first loop in the region providing the electrical contact.

The invention therefore relates to an assembly, comprising:
- a temperature sensor comprising a printed circuit board supporting a temperature-sensitive component;
- a conductive yarn having an end folded to form a first loop in a region providing electrical contact with the printed circuit board; and
- an insulating member placed around the first loop in the region providing the electrical contact.

In an embodiment, the conductive yarn may be covered by an insulating coating layer, removed at the end folded to form the first loop.

In an embodiment, the assembly may further comprise a conductive wire having a first end connected to the printed circuit board and a second end folded to form a second loop, wherein the first and second loops may be interlocked in the region providing the electrical contact.

In an embodiment, the insulating member may comprise a thermoretractable sleeve placed around the mutually interlocked first and second loops.

In an embodiment, the printed circuit board may have a conductive edge portion, wherein a hole may be provided in the conductive edge portion, and wherein the first loop of the conductive yarn may be passed through the hole.

In an embodiment, the conductive yarn may be tied in the hole.

In an embodiment, the first loop of the conductive yarn and the hole may be encapsulated by the insulating member.

In an embodiment, the temperature sensor may further comprise a housing receiving at least the printed circuit board and the temperature-sensitive component.

In an embodiment, the conductive yarn may comprise an arrangement of chain stitches.

In an embodiment, the conductive yarn may be sewn to a flexible substrate.

In an embodiment, the conductive yarn may have a connection portion extending beyond the flexible substrate, wherein the connection portion may be folded to form the first loop.

Another aspect of the disclosure is related to a wearable article, comprising at least:
- an electronic board;
- an assembly of the invention.

According to one specific embodiment, the invention relates to a wearable article, comprising at least:
- an electronic board;
- an assembly as disclosed above, comprising a temperature sensor comprising a printed circuit board supporting a temperature-sensitive component and a conductive yarn having an end folded to form a first loop in a region providing electrical contact with the printed circuit board.

The wearable article may be configured to press the temperature sensor against a body of a user wearing the wearable article, in such a way that temperature variation of the body of the user is detected by electronic board.

According to another specific embodiment, the invention relates to a wearable article, comprising at least:
- an electronic board;
- an assembly as disclosed above, comprising at least one conductive yarn and at least one elongation sensor, each conductive yarn being knotted to one of the at least one elongation sensor, wherein each conductive yarn is connected to the electronic board.

The wearable article may be configured to press each elongation sensor against a body of a user wearing the wearable article, in such a way that a length of each elongation sensor varies when the user breathes or when muscles of the user are contracted or relaxed.

The electronic board may be configured to detect variations of electrically conductive properties of the elongation sensor and deduce an elongation of the elongation sensor from it. For example, breathing amplitude or breathing frequency of a user wearing the wearable article may be deduced.

In an embodiment, the wearable article may be a garment and comprise a flexible support covering a portion of a body of the user. The garment may refer to an item of clothing or apparel. The garment may be a top. The top may be a shirt, t-shirt, blouse, sweater, dress, underwear, athletic clothing, swimwear, belt, jacket/coat, or vest.

The flexible support may be a clothing material, for example a non-conductive, flexible fabric. The flexible support may be stretched when worn by a user and press the elongation sensor and/or temperature sensor against the body of the user, in such a way that a length of the elongation sensor varies when the user breathes or when muscles of the user are contracted or relaxed and/or temperature is measured.

The wearable article may be a brassiere or a T-Shirt.

The integration of elongation sensors into a T-Shirt or a brassiere allows studying breathing parameters or muscle contraction/relaxation over many hours, as long as the T-Shirt or brassiere is worn.

In an embodiment, the wearable article may further comprise at least one elastic belt. Each elongation sensor may be attached to one of the at least one elastic belt.

The elastic belt may allow attaching the elongation sensor to the body of the user. The elastic belt may press the elongation sensor against the body of the user wearing the wearable article, in such a way that a length of the elongation sensor varies when the user breathes or when muscles of the user are contracted or relaxed.

In an embodiment, the elongation sensor may have a loop portion at a distance from the electronic board. The elongation sensor may have a first section extending from a first knotted portion to the loop portion and a second section extending from the loop portion to a second knotted portion

The first and second sections are portions of the elongation sensor extending away from the electronic board, starting from the first and second knotted portions, where the elongation sensor is indirectly connected to the electronic board by the conductive yarn.

In the first knotted portion, the elongation sensor is knotted to a first conductive yarn that is connected to the electronic board. In the second knotted portion, the elongation sensor is knotted to a second conductive yarn that is connected to the electronic board.

The elongation sensor may extend, starting from a point where it is indirectly connected to the electronic board, away from the electronic board, then form a loop, and extend further back to the electronic board to a point where it is indirectly connected to the electronic board. The elongation sensor may be "U"-shaped.

This configuration of the elongation sensor may be useful in a wearable article when the elongation sensor is intended to surround only a portion of the circumference of a body part of the user.

In an embodiment, each elongation sensor may be disposed to be located around at least part of an abdomen and/or a chest of a user wearing the wearable article.

During breathing, the change of the circumference of the abdomen and the chest may be more important when compared to other body parts. Elongation sensors located around the chest/the abdomen allow precise determination of breathing parameters of a user, such as breathing amplitude or breathing frequency.

In an embodiment, the wearable article may further comprise a waterproof housing configured to receive the electronic board. The elastic belt may be attached to the waterproof housing.

In an embodiment, the wearable article may be in the form of a garment further comprising a flexible support connected at least to the waterproof housing.

In an embodiment, the waterproof housing may comprise an orifice. The conductive yarn may pass from the region providing electrical contact to the electronic board through an orifice in the waterproof housing.

In an embodiment, the orifice may be sealed by elastic tape.

### BRIEF DESCRIPTION OF THE DRAWINGS

The accompanying drawings, which are incorporated herein and form a part of the specification, illustrate the present invention and, together with the description, further serve to explain the features of the invention and to enable a person skilled in the pertinent art to make and use the invention. In the drawings, like reference characters indicate identical or functionally similar elements.
- Figure 1 is a schematic representation of the front side of an example of a wearable article according to the invention, the wearable article being a brassiere comprising two elongation sensors;
- Figure 2 is a schematic representation of the front side of another example of a wearable article according to the invention, the wearable article being a T-Shirt comprising an elongation sensor;
- Figure 3 is a schematic representation of the back side of the T-Shirt of Fig. 2;
- Figure 4 is a schematic representation of an arrangement of the elongation sensor, of two conductive yarns, and of two triple knots formed by each of the two conductive yarns with the elongation sensor in the T-Shirt of Fig. 2 and Fig. 3;
- Figure 5 is a partial, schematic cross-sectional view of an example of the T-Shirt of Fig. 2;
- Figures 6-12 are photographs illustrating respectively different steps of an assembling to a triple knot of an elongation sensor and a conductive yarn in a wearable article according to the invention;
- Figure 13 is an isolated schematic representation of a triple knot of an elongation sensor and a conductive yarn of an assembly according to the invention;
- Figure 14 is an isolated schematic representation of a conductive yarn sewn in chain stitches to a flexible substrate in an assembly according to the invention.
- Figure 15 is a schematic representation of another example of a wearable article according to the invention, the wearable article being a brassiere comprising two temperature sensors;
- Figure 16 is schematic representation of an embodiment of an assembly of a temperature sensor and a conductive yarn.
- Figure 17 is schematic representation of another embodiment of an assembly of a temperature sensor and a conductive yarn.

### DESCRIPTION OF PREFERRED EMBODIMENTS

According to one embodiment, the assembly of the invention relates to an assembly of an elongation sensor and a conductive yarn.

Now, a wearable article that includes physiological sensors for detecting physiological signals of the body of a user wearing the wearable article will be described.

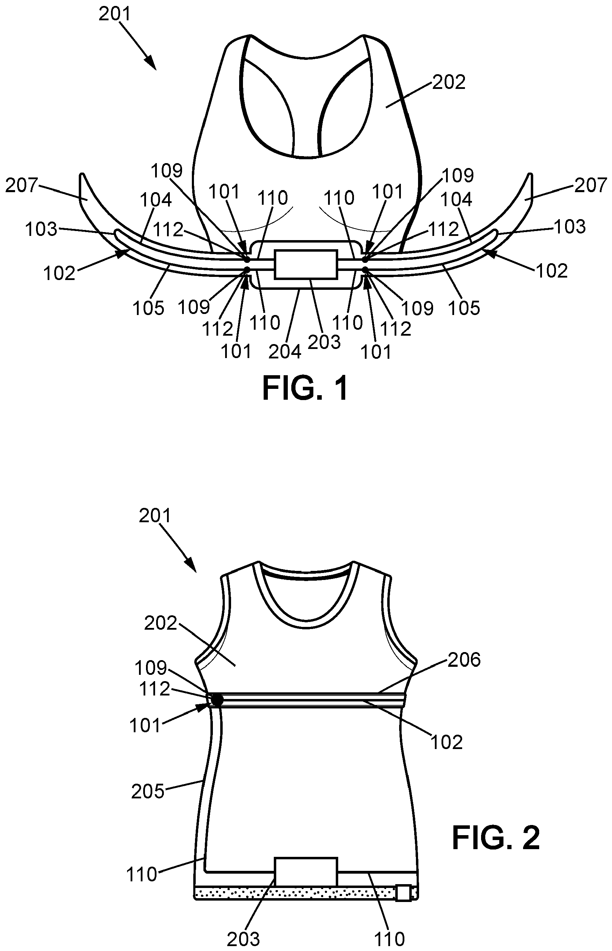

In the non-limiting embodiment shown in figure 1, the wearable article 201 is a brassiere that includes sensors to detect signals related to heart activity, breathing or body temperature of a user.

The brassiere comprises two elongations sensors 102 and four conductive yarns 110. Each conductive yarn 110 is assembled with one of the four elongation sensors 102 to form a respective assembly 101.

Each elongation sensor 102, which may also be called breathing wire, is a flexible yarn that comprises a body of flexible material such as silicone with conductive particles embedded therein, for example carbon particles. Each elongation sensor 102 has electrically conductive properties that vary as a function of elongation of the elongation sensor 102, i.e. when the user breathes. In this example, each elongation sensor 102 further has a protective cover made from Solvron^{®} threads wrapped around the body of flexible material. The protective cover facilitates manipulation of the elongation sensor 102.

Such an elongation sensor 102 typically has a resistance of the order of kΩ/m, for example between 100 kΩ/m - 200 kΩ/m.

An elongation sensor that may be used in the wearable article 201 has in a rest state a resistance of 136 kΩ/m when 5 g of carbon particles are used in relation to 1000 g of silicone. Another elongation sensor has in a rest state a resistance of 165 kΩ/m when 50 g of carbon particles are used in relation to 1000 g of silicone.

The brassiere further comprises a flexible support 202 covering parts of the chest and the back of the user, and further the shoulders in the form of shoulder straps. The brassiere comprises a waterproof housing 204 that receives an electronic board 203 configured to collect and process signals obtained from the elongation sensors 102.

The waterproof housing 204 is made from polyester plates that are soldered to each other on their respective edges. According to special embodiment, the waterproof housing 204 may be as disclosed in WO2021028223.

The brassiere also comprises two elastic belts 207, each elongation sensor 102 being attached to an elastic belt 207. The waterproof housing 204 is connected to the two elastic belts 207 that are attached to opposite sides of the waterproof housing 204. When the brassiere is worn, the waterproof housing 204 is located at the sternum of the user, and the elastic belts 207 extend around a part of the torso of the user. The elastic belts 207 can be linked to each other by the user at the back of the user.

The flexible support 202 of the brassiere is connected to the waterproof housing 204. Therefore, a bottom portion of the flexible support 202 of the brassiere is folded and sewn in order to create a pocket (not shown in figure 1 for clarity reasons) that receives the waterproof housing 204. A portion of the elastic belts 207 protrudes from the pocket in opposite directions.

Each elongation sensor 102 is connected to the electronic board 203 indirectly by conductive yarn 110. More precisely, two conductive yarns 110 per elongation sensor 102 are used to connect each elongation sensor 102 indirectly to the electronic board. The conductive yarns are sewn to a conductive edge portion with holes of the electronic board 203.

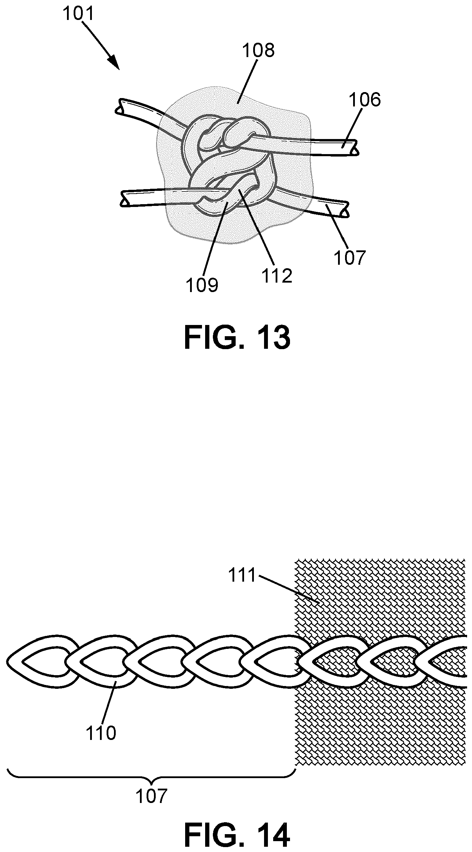

Each conductive yarn 110 has a connection portion 107 (described in more detail in relation to figure 14) that is intended to be assembled with a corresponding connection portion of an elongation sensor.

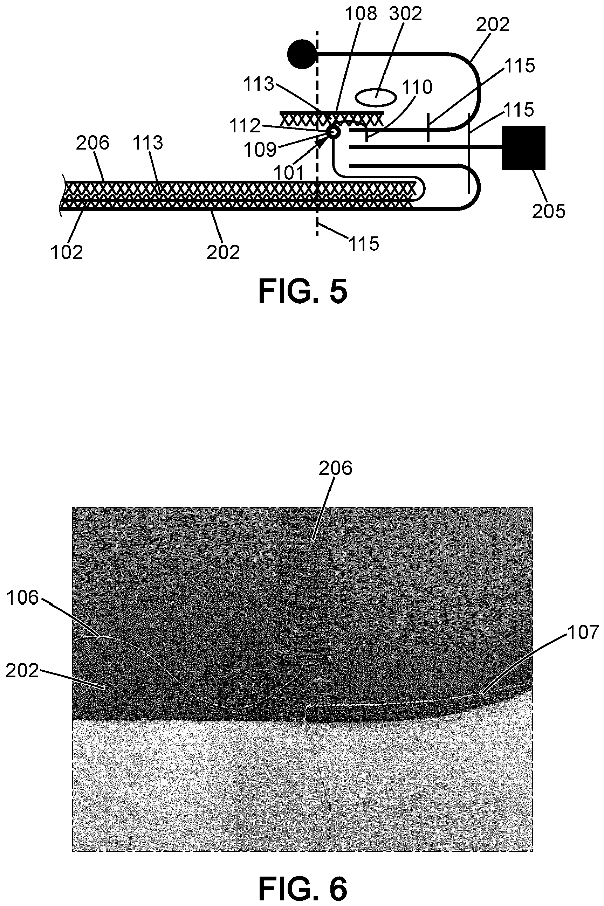

A first connection portion of an elongation sensor 102 is knotted to a corresponding connection portion 107 of one conductive yarn 110 in a knotted portion 112, as will be described in detail in relation to figures 6 -13. A second connection portion of the same elongation sensor 102 is knotted to a corresponding connection portion 107 of another conductive yarn 110 in another knotted portion 112.

Each knotted portion 112 ties the elongation sensor 102 to the conductive yarn 110. The elongation sensor 102 and the conductive yarn 110 are held tight to each other in the knotted portion 112.

Each knotted portion 112 creates an electrical connection between the elongation sensor 102 and the conductive yarn 110. Each knotted portion 112 comprises a triple knot 109, as will be described in relation to figures 6-13. The brassiere of figure 1 comprises four such triple knots 109 (two triple knots 109 for each of the two elongation sensors 102).

In this example, each conductive yarn 110 is built from two assembled *Circuitex* silver yarns from the company *Noble Biomaterials.* Each *Circuitex* resistive element has a flexible polyamide core plated with silver. Each conductive yarn 110 corresponding to the two assembled *Circuitex* silver yarns has a resistance of 172 Ω/m. Each conductive yarn 110 is further covered by two *Solvron*^{®} threads to reinforce the conductive yarn 110 and to limit its unravelling.

In this example, each elongation sensor 102 forms a loop portion 103 at a distance from the electronic board 203, i.e. the elongation sensor 102 is "U"-shaped. More precisely, a first section 104 of the elongation sensor 102 extends from a first triple knot formed with the conductive yarn 110 to the loop portion 103, and a second section 105 extends from the loop portion 103 back to a second triple knot formed with the conductive yarn 110.

The loop portions 103 of the elongation sensors 102 are located in the belts 207. In order to protect the elongation sensors 102 against water and wear, the elongation sensors 102 are encapsulated by elastic straps attached to a respective elongation sensor 102 by use of an adhesive material. An elastic, adhesive material is placed between the elastic straps that surrounds the respective elongation sensors 102 and provides a flexible matrix in which the respective elongation sensor 102 is embedded. The encapsulation of the elongation sensor 102 is created by heat-bonding.

When the belts 207 are tightened to the torso of the user, the elastic belts 207 press the elongation sensors 102 against the torso, such that a length of the elongation sensor 102 varies when the user breathes or when muscles under the elongation sensor 102 are contracted or relaxed.

Having two elongation sensors 102 in the brassiere rather than one single elongation sensor 102 is advantageous in particular when the user wearing the brassiere lays on one side. One elongation sensor 102 may then be squeezed and not work properly. However, the other elongation sensor 102 is still functional.

The electrical connection path from the elongation sensors 102 to the electronic board 203 has a waterproof encapsulation. Therefore, the conductive yarn 110 is encapsulated by elastic tape attached to the conductive yarn 110 by heat-bonding.

The electronic board 203 and a portion of the conductive yarn 110 connected to the electronic board 203 is encapsulated by a silicone matrix in order to avoid intrusion of water into the electronic board 203. The encapsulation by a silicone matrix provides an additional protection for the electronic board 203, in addition to the placing of the electronic board 203 in the waterproof housing 204.

The conductive yarn 110 passes from the electronic board 203 through small orifices in the polyester plates of the waterproof housing 204 and further to the elongation sensor 102. The orifices in the polyester plates are sealed by elastic tape attached to the polyester plates by heat-bonding, in order to ensure that the space enclosed by the polyester plates is waterproof.

In another non-limiting embodiment shown in figures 2, 3 and 5, the wearable article 201 is a T-Shirt that includes sensors to detect signals related to heart activity, breathing or body temperature of a user.

The T-Shirt comprises one elongation sensor 102 surrounding the chest of a user. In particular, the T-Shirt comprises an assembly 101 of an elongation sensor 102 and two conductive yarns 110.

The T-Shirt comprises a flexible support 202 having elasticity and covering the chest, back, abdomen and upper arms of a user. The flexible support 202 is stretched when worn by a user, thus pressing the elongation sensor 102 of the T-Shirt against the body of the user such that a length of the elongation sensor 102 varies when the user breathes or when muscles of the user are contracted or relaxed.

The T-Shirt further comprises a lateral zipper 205, allowing opening and closing the T-Shirt. The lateral zipper 205 makes it easy for a user to put the T-Shirt on and off.

The T-Shirt further comprises an electronic board 203 configured to collect and process signals obtained from the elongation sensors 102. The electronic board 203 is located in the region of the navel of a user.

The elongation sensor 102 and the conductive yarn are assembled in the same way as in the brassiere of figure 1 and as will be further described in relation to figures 6 - 13.

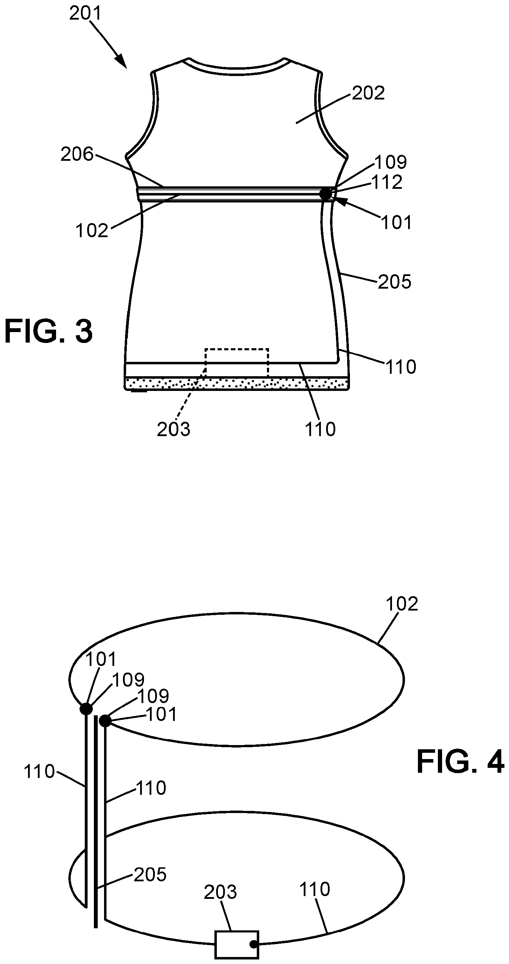

The specific example of figures 2 and 3 and in particular of figure 4 illustrate the path of the elongation sensor 102 and of the two conductive yarns 110 in the T-Shirt. The connection portion 107 of each of these two conductive yarns 110 are attached to opposite sides of the electronic board 203. The conductive yarns 110 are guided from the electronic board 203 in opposite directions parallel to the bottom edge of the T-Shirt up to the zipper 205. Thus, the conductive yarn 110 almost entirely surrounds the abdomen. Both conductive yarns 110 are then guided along the zipper 205 (and on opposite sides of the zipper 205) up to the chest region. The conductive yarns 110 are attached to the flexible support 202 of the T-Shirt along its path from the electronic board 203 to the elongation sensor 102.

A connection portion 107 of each of the two conductive yarns 110 is knotted in the chest region close to the zipper 205 to respective connection portions of the elongation sensor 102 in the knotted portion 112. Thus, the elongation sensor 102 is connected to one conductive yarn 110 close to one side of the zipper 205, surrounds almost entirely the chest and is connected to another conductive yarn 110 close to the other side of the zipper 205.

The knotted portion 112 comprises a triple knot 109, as will be described in relation to figures 6 - 13. Thus, each elongation sensor 102 forms one triple knot 109 with one conductive yarn 110 and another triple knot 109 with another conductive yarn 110. The T-Shirt of figures 2 and 3 comprises two such triple knots 109.

A similar arrangement not shown in the figures is provided for the elongation sensor 102 surrounding the abdomen of the user.

In the specific example of the T-Shirt shown in figure 5, stitches 115 are used in the T-Shirt to fix the elongation sensor to the flexible support 202 in a region close to the knotted portion 112, in order to reduce forces acting on the knotted portion 112 when the elongation sensor 102 is stretched. Furthermore, other sensors such as a temperature sensor 302 may be included into the T-Shirt.

A skilled person would understand that a T-Shirt without a zipper is within the scope of the disclosure. When no zipper is present, the elongation sensors 102 surround the chest (or the abdomen) of the user and are indirectly connected to the electronic board 203 by conductive yarn 110. In this example, the conductive yarn 110 does not surround the abdomen.

The electrical connection path from the elongation sensors 102 to the electronic board 203 has a waterproof encapsulation.

In particular, as shown in figure 5, the elongation sensor 102 is encapsulated by the flexible support 202 of the T-Shirt and an elastic strap 206 adhered to the inner side of the T-Shirt by heat-bonding. An adhesive material 113 placed between the elastic strap 206 and the flexible support 202 covers the elongation sensor 102 and ensures adhesion of the flexible support 202, the elastic strap 206 and the elongation sensor 102. The adhesive material 113 provides a flexible matrix in which the elongation sensor 102 is embedded.

The elastic strap 206 has elasticity and is pre-stressed, for example by 20%, when the T-Shirt is worn and with respect to a rest position of the elastic strap 206 when the T-Shirt is not worn. Thus, the elongation sensor 102 is pressed against the body of the user when the user wears the T-Shirt.

The electronic board 203 and a portion of the conductive yarn 110 connected to the electronic board 203 are encapsulated by a silicone matrix in order to avoid intrusion of water into the electronic board 203.

A method for assembling the connection portion 106 of the elongation sensor 102 and the connection portion 107 of the conductive yarn 110 in a knotted portion 112 is shown in figures 6 to 13, for the case of the elongation sensor 102 surrounding the chest of the user wearing the T-Shirt. The method may be applied in the same way in the brassiere.

The connection portions 106, 107 before assembling are shown in figure 6. The connection portion 106 of the elongation sensor 102 has a length of at least 6 cm and protrudes from the elastic strap 206 attached to the flexible support 202. The conductive yarn 110 is attached to the flexible support 202 and has a connection portion 107 of a length of at least 6 cm not attached to the flexible support 202.

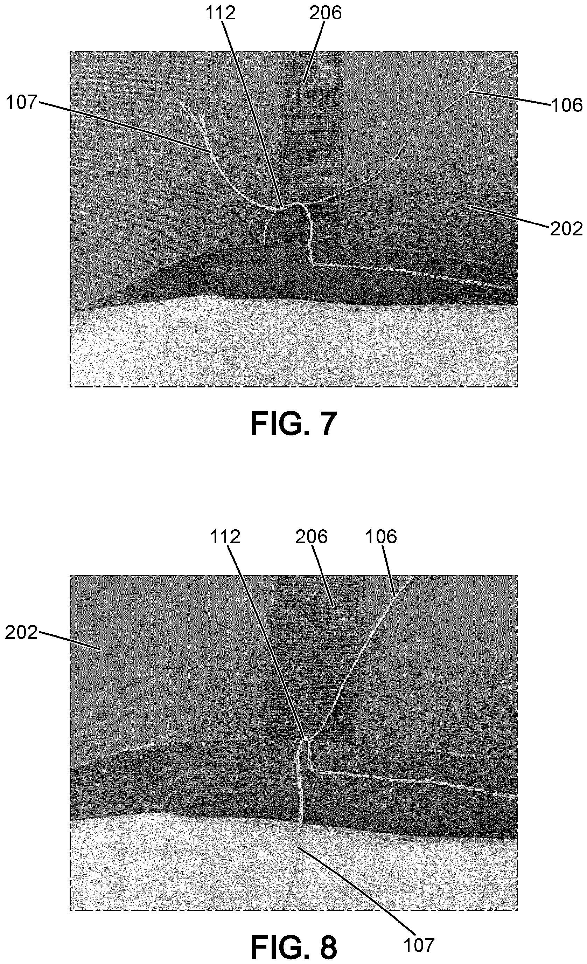

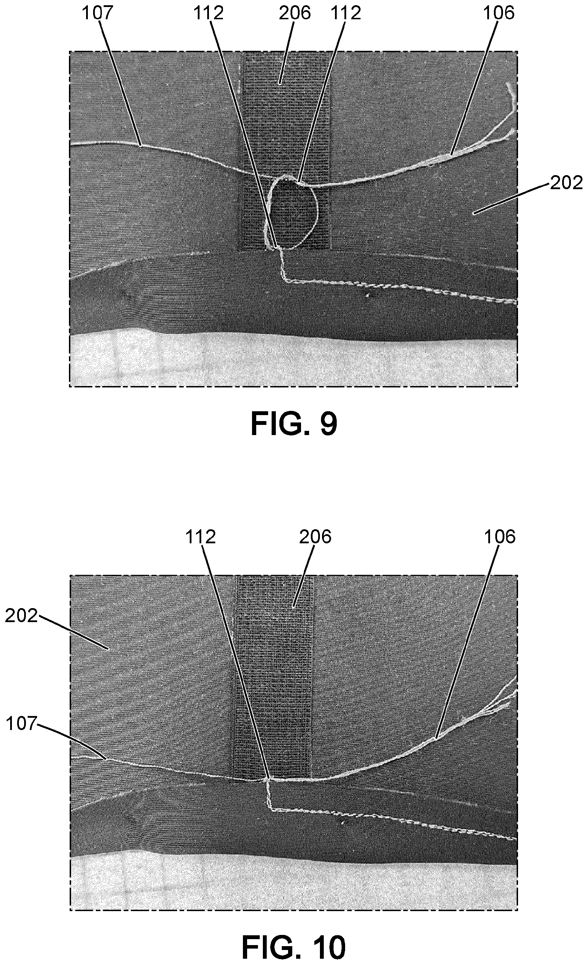

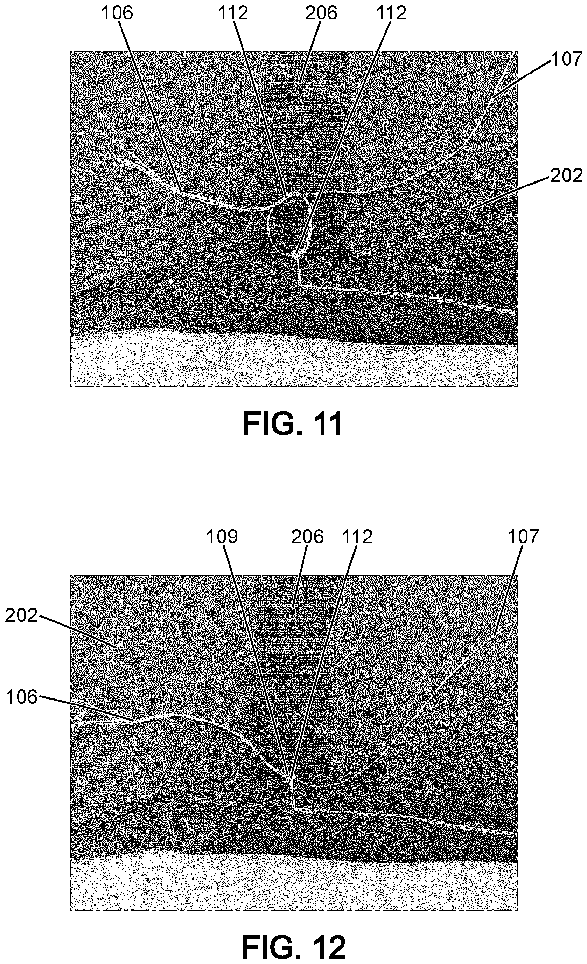

A first knot is made by crossing the connection portion 106 of the elongation sensor 102 under the connection portion 107 of the conductive yarn 110 (figure 7), like when starting to tie shoelaces. The knot is then tightened (figure 8). A second knot is made above the first knot by crossing the remaining portion of the connection portion 106 of the elongation sensor 102 under the remaining portion of the connection portion 107 of the conductive yarn 110 (figure 9), and by pulling the elongation sensor 102 and the conductive yarn 110 tight (figure 10). A third knot is made above the second knot in the same way that the second knot was made above the first knot (figures 11, 12). Figure 12 shows the resulting knotted portion 112, which is a triple knot 109.

A schematic representation of the resulting knotted portion 112 of figure 12 is shown in figure 13. The knotted portion 112 is a triple knot 109, i.e. it comprises three superposed individual knots formed one above the other. The triple knot 109 is stabilized and protected against water by covering it with an isolating wrapping layer 108.

For example, one individual knot may be relatively unstable and become loose over time. On the contrary, three superposed knots, i.e. the triple knot 109, provides a stable connection between the elongation sensor 102 and the conductive yarn 110, even when the elongation sensor 102 and the conductive yarn 110 are made from materials that do not adhere to each other, and even when forces are applied to the elongation sensor 102.

An adhesive material 113 is used when attaching the isolating wrapping layer 108 to the knotted portion 112, as is also illustrated in figure 4 showing the cross-sectional view of the T-Shirt. The isolating wrapping layer 108 may be attached to the knotted portion 112 by heat-bonding.

Figure 14 shows a specific embodiment of the conductive yarn 110. The conductive yarn 110 is, in this example, sewn in chain stitches to a flexible substrate 111 such as tulle. An arrangement of chain stitches extends beyond the flexible substrate 111 by 6 cm or more and forms the connection portion 107 of the conductive yarn 110.

According to another embodiment, the assembly of the invention relates to an assembly of a temperature sensor and a conductive yarn.

The assembly of the invention can be comprised in a wearable article 201 that includes temperature sensors for detecting temperature-related signals of the body of a wearer.

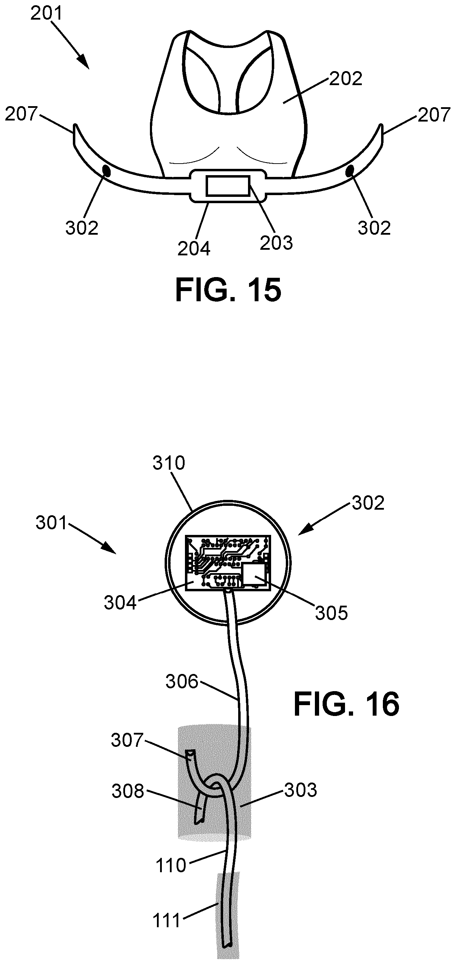

The example of figure 15 shows a brassiere that comprises two temperature sensors 302 adapted to detect the temperature of the body of a wearer.

The brassiere may comprise a flexible support 202 covering parts of the chest and the back of a wearer, and further the shoulders in the form of shoulder straps. The flexible support 202 may be made of non-conductive fabric, like cotton/elastane or polyamide/elastane. The flexible support may contain between 6% and 12% of elastane such that the wearable article tightly fits the wearer's body. This makes sure that the sensors included in the wearable article are in close contact with the wearer's skin.

The brassiere may further comprise a waterproof housing 204 located at the sternum of the wearer. The waterproof housing 204 may receive an electronic board 203 configured to collect and process signals obtained from the temperature sensors 302. The electronic board 203 may be coupled to a battery.

The waterproof housing 204 may be made from polyester plates that are soldered to each other on their respective edges. According to special embodiment, the waterproof housing 204 may be as disclosed in WO2021028223.

The waterproof housing 204 may be connected to two elastic belts 207 that are attached to opposite sides of the waterproof housing 204. When the brassiere is worn, the elastic belts 207 may extend around a part of the torso of the wearer and be linked to each other at the back of the wearer.

The flexible support of the brassiere may be connected to the waterproof housing 204. Therefore, a bottom portion of the flexible support of the brassiere may be folded and sewn in order to create a pocket (not shown in figure 15) that receives the waterproof housing 204. A portion of the elastic belts 207 may protrude from the pocket in opposite directions.

Each belt 207 may have at least one temperature sensor 302 mounted on it, allowing temperature measurements at two positions of the body of the wearer.

The temperature sensors 302 may be located at any suitable position on the inner side of the elastic belts 207, i.e. on the side of the elastic belts 207 intended to be in contact with the skin of the wearer. When tightened to the torso of the wearer, the elastic belts 207 press the temperature sensors 302 against the torso.

Alternatively, the temperature sensor may be located at any other position on the wearable article.

In some embodiment, flexible support 202 comprises openings allowing temperature sensors 302 to be in direct contact with the skin of the wearer.

In some embodiment, temperature sensors 302 are in indirect contact with the skin of the wearer e.g. thanks to metallic part, for example snap button.

The temperature sensors 302 may be electrically connected to the electronic board 203 by use of conductive yarn 110.

To this effect, the electronic board 203 may be disposed on a tulle support and comprise a conductive edge portion with holes. The conductive yarn 110 may be sewn to the tulle support and through one or more holes.

Since the temperature sensors 302 are located on the elastic belts 207 rather than at other positions in the brassiere such as the shoulder area, distances between the temperature sensors 302 and the electronic board 203 are short, and short conductive yarns 110 may be used for connection of the temperature sensors 302 and the electronic board 203.

The electrical connection path from the temperature sensors 302 to the electronic board 203 may have a waterproof encapsulation.

Therefore, the conductive yarn 110 may be covered by encapsulating it with elastic tape that is affixed to the conductive yarn 110 by heat bonding.

The electronic board 203 and the portion of the conductive yarn 110 connected to the electronic board 203 may be encapsulated by a silicone matrix in order to avoid intrusion of water into the electronic board 203. The encapsulation by a silicone matrix provides an additional protection for the electronic board 203, in addition to the placing of the electronic board 203 in the waterproof housing 204.

The encapsulated conductive yarn 110 may pass from the electronic board 203 through small orifices in the polyester plates of the waterproof housing 204 and further to the temperature sensors 302. One or more conductive yarns 110 may be guided through the same orifice. The orifices in the polyester plates may be sealed by elastic tape affixed to the polyester plates by heat bonding, in order to ensure that the space enclosed by the polyester plates is waterproof.

The temperature sensors 302 may have a waterproof encapsulation as well, as will be explained below.

In another non-limiting embodiment not shown in the figures, the wearable article may be a T-Shirt that includes sensors to detect signals related to heart activity, breathing or body temperature of a wearer. In particular, the T-Shirt may comprise several temperature sensors.

The T-Shirt may comprise a flexible support covering the chest, back, abdomen and upper arms of a wearer. The T-Shirt may further comprise an electronic board configured to collect and process signals obtained from the temperature sensors. The electronic board may be encapsulated by a silicone matrix in order to avoid intrusion of water into the electronic board. In addition, each temperature sensor may have a waterproof encapsulation.

The temperature sensors 302 may be located at any suitable position on the inner side of the T-Shirt, i.e. on the side of the T-Shirt oriented towards the skin of a wearer. The flexible support of the T-Shirt may have elasticity and press the temperature sensors against the skin of a wearer.

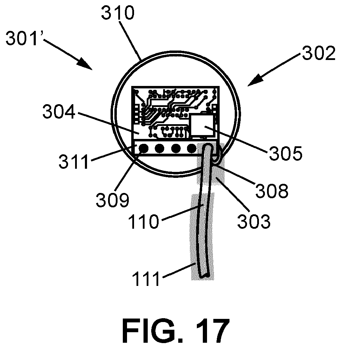

The temperature sensors may be connected to the electronic board by conductive yarn affixed to the inner side of the T-Shirt. The conductive yarn may have a waterproof encapsulation. Examples of an assembly 301 and 301' of a temperature sensor 302 and a conductive yarn 110 according to the invention are shown in figures 16 and 17, respectively.

Each temperature sensor 302 comprises a temperature-sensitive component 305 that is soldered on a printed circuit board 304.

The temperature-sensitive component 305 may be a thermistor, in particular a negative temperature coefficient (NTC) thermistor whose resistance decreases with temperature.

In the first embodiment of the assembly 301 shown in figure 16, an electrical contact between the temperature sensor 302 and the conductive yarn 110 is established indirectly by means of a conductive wire 306, for example a multifilament copper wire.

The conductive wire 306 has a first end soldered to the printed circuit board 304 and a second end connected to the conductive yarn 110. Therefore, an end of the conductive yarn 110 serving as connection portion 107 may be folded and form a "U-shaped" loop 308, referred to as "first loop" hereafter. In a similar manner, the second end of the conductive wire 306 that is not soldered to the printed circuit board 304 may be folded and form a "U-shaped" loop 307, referred to as "second loop" hereafter.

When the "U-shaped" loops 307, 308 are oriented in opposite directions with respect to each other they can engage and interlock, similarly to two adjacent elements of a chain.

The connection portion 107 of the conductive yarn 110 forming the first loop 308 may be without the above-mentioned elastic tape, in order to establish an electrical contact with the conductive wire 306.

An insulating member 303 in the form of a thermo-retractable sleeve may be disposed around the interlocked loops 307, 308. For example, a shrinkable plastic tube may be fitted on the interlocked loops 307, 308 and shrunk by the application of heat, thus stabilizing the connection portion 107 and protecting it against humidity.

In the second embodiment of the assembly 301' shown in figure 17, no conductive wire 306 is used. Instead, an electrical contact between the temperature sensor 302 and the conductive yarn 110 is established by connecting the conductive yarn 110 directly to the printed circuit board 304. Therefore, the printed circuit board 304 may be provided with a conductive edge portion 311 with holes 309 in it. One end of the conductive yarn 110 may be tied to the holes 309.

The connection portion 107 of the conductive yarn 110 tied to the holes 309 may be without the above-mentioned elastic tape, in order to establish an electrical contact with the conductive edge portion 311.

The region in which the conductive yarn 110 is tied to the printed circuit board 304 may by encapsulated with an insulating member comprising an insulating material 303 in order to create a waterproof protection. In an embodiment, the insulating material may be attached by heat-bonding. Heat-bonding allows assembling different materials by applying heat and pressure to them. In another embodiment, the insulating material consists in plastic housing.

In both embodiments of the assembly 301, 301', the temperature sensor 302 may be placed in a housing 310, for example a plastic housing that provides a waterproof protection for the components in the housing 310. The plastic housing may be composed of two or more individual plastic plates that are glued one to another. The housing 310 may further be filled with a filling material such as epoxy resin.

The plastic plates may have high thermal conductivity, allowing fast conduction of heat and fast detection of temperature variations of the body of a user.

The conductive wire 306 (first embodiment) or the conductive yarn 110 (second embodiment) connected to the printed circuit board protrude from the housing 310 in order to enable connection of the temperature-sensor located in the housing 310 to the electronic board 203.

A possible embodiment of the conductive yarn 110 is shown in figure 14.

The conductive yarn 110 may be sewn in chain stitches to a flexible substrate 111 such as tulle. A portion of the flexible substrate 111 may then be removed, such that an arrangement of chain stitches extends beyond the flexible substrate 111 and forms the above-mentioned connection portion 107 of the conductive yarn 110 that may then interlocked with the conductive wire 306 (first embodiment shown in figure 16) or be tied to the holes 309 of the conductive edge portion 311 of the printed circuit board 304 (second embodiment shown in figure 17).

Compared to a conductive yarn 110 in a linear arrangement, the arrangement of a conductive yarn 110 in chain stitches is more resilient and easier to manipulate.

It will be appreciated that the embodiments described above are illustrative of the invention disclosed herein and that various modifications can be made without departing from the scope as defined in the appended claims.

## Claims

1. An assembly (101) comprising at least:
- a conductive yarn (110); and
- an elongation sensor (102) having electrically conductive properties,
wherein the conductive yarn (110) is knotted to the elongation sensor (102) in a knotted portion (112) to provide electrical contact between the conductive yarn (110) and the elongation sensor (102).

2. The assembly of claim 1, further comprising an isolating wrapping layer (108) covering the knotted portion (112).

3. The assembly of claim 2, wherein the isolating wrapping layer (108) is attached to the knotted portion (112) by heat-bonding.

4. The assembly of any one of the preceding claims, wherein the knotted portion (112) comprises a triple knot (109).

5. The assembly of any one of the preceding claims, wherein the elongation sensor (102) comprises a body of flexible material and conductive particles embedded in the body of flexible material.

6. The assembly of any one of the preceding claims, wherein the conductive yarn (110) comprises an arrangement of chain stitches.

7. The assembly of any one of the preceding claims, wherein the assembly comprises a flexible substrate (111), and wherein the conductive yarn (110) is sewn to the flexible substrate (111).

8. The assembly of claim 7, wherein the conductive yarn (110) comprises a connection portion (107) extending beyond the flexible substrate (111), and wherein the connection portion (107) of the conductive yarn (110) is knotted to the elongation sensor (102) in the knotted portion (112).

9. A wearable article (201), comprising at least:
- an electronic board (203);
- an assembly (101) as claimed in any one of the preceding claims, comprising at least one conductive yarn (110) and at least one elongation sensor (102), each conductive yarn (110) being knotted to one of the at least one elongation sensor (102), wherein each conductive yarn (110) is connected to the electronic board (203);
wherein the wearable article (201) is configured to press each elongation sensor (102) against a body of a user wearing the wearable article (201), in such a way that a length of each elongation sensor (102) varies when the user breathes or when muscles of the user are contracted or relaxed.

10. The wearable article of claim 9, wherein the wearable article (201) is a garment and comprises a flexible support (202) covering a portion of a body of the user.

11. The wearable article of claim 10, wherein the wearable article (201) is a brassiere.

12. The wearable article of claim 10, wherein the wearable article (201) is a T-Shirt.

13. The wearable article of any one of claims 9 to 12, further comprising at least one elastic belt (207), wherein each elongation sensor (102) is attached to one of the at least one elastic belt (207).

14. The wearable article of any one of claims 9 to 13, wherein the elongation sensor (102) has a loop portion (103) at a distance from the electronic board (203), a first section (104) extending from a first knotted portion (112) to the loop portion (103) and a second section (105) extending from the loop portion (103) to a second knotted portion (112).

15. The wearable article of any one of claims 9 to 14, wherein each elongation sensor (102) is disposed to be located around at least part of an abdomen and/or a chest of a user wearing the wearable article (201).
